(19) Europäisches Patentamt
European Patent Office
Office européen des brevets

(11) Publication number: **0 483 426 A1**

# (12) EUROPEAN PATENT APPLICATION

(21) Application number: **90311982.4**

(51) Int. Cl.$^5$: **A61K 7/32**, A61L 9/01

(22) Date of filing: **01.11.90**

(43) Date of publication of application:
**06.05.92 Bulletin 92/19**

(84) Designated Contracting States:
**AT BE CH DE DK ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Chatterjee, Ranjit (NMN)**
**11041 Sycamore Grove Lane**
**Cincinnati, Ohio 45241(US)**

(74) Representative: **Brooks, Maxim Courtney et al**
**Procter & Gamble (NTC) Limited Whitley Road Longbenton**
**Newcastle-upon-Tyne NE12 9TS(GB)**

## (54) Thiol heterocyclic deodorant composition and method of deodorization.

(57) The present invention provides compositions containing a thiol heterocyclic compound and a topical carrier for topical application to the skin in the axilla or crotch region or to articles of clothing which are worn in the vicinity of such skin. The applicable deodorant agents comprise thiol heterocyclic compounds, or a pharmaceutically-acceptable salt thereof, comprising a heterocyclic ring structure having at least one heteroatom in the ring structure selected from the group consisting of oxygen, nitrogen, and sulfur; and wherein said heterocyclic ring structure has a least 1 thiol substituent attached to a carbon atom of said heterocyclic ring.

Methods for controlling malodor comprising depositing onto skin in the axilla or crotch region, or to articles of clothing worn in the vicinity of the skin of a safe and effective amount of a thiol heterocyclic deodorant agent of the present invention are also provided.

EP 0 483 426 A1

## TECHNICAL FIELD

The present invention related to compositions and methods for the treatment or prevention of malodor associated with human perspiration.

## BACKGROUND OF THE INVENTION

Vast volumes of the chemical, medical and cosmetic literature have been generated concerning the causes, effects and prevention of human perspiration. "Perspiration", or "sweat", may be generally defined as including the excretion of the sweat glands situated in the corium or subcutaneous tissue, known as eccrine sweat glands, distributed over most of the body surface. While perspiration serves an important function in cooling the body through its evaporation, the by-products resulting from its bacterial degradation may be malodorous and aesthetically objectionable.

Malodor is particularly associated with perspiration secreted at areas of the body where apocrine sweat glands, in addition to eccrine sweat glands, can be found. Two principal areas which have apocrine sweat glands are the axilla and the crotch. See, for example, J. Labows, et al., "Perspectives on Axillary Odor", 34 J. Soc. Cosmetic Chemists 193-202 (1982) and P. Jackman, "Body Odor - The Role of Skin Bacteria" 1 Seminars in Dermatology 143-148 (1982). A variety of bacteria have been implicated in producing axillary malodor, a principal bacteria responsible for such malodor being the gram positive microflora naturally found in the axilla, e.g., the diphtheroids, such as the Corynebacteria and Propionibacteria, and the gram positive cocci, such as the Staphylococci and Micrococci.

A great number of compositions have been developed and described in the literature for reducing or eliminating the aesthetic problems associated with perspiration. See, for example, S. Plechner, "Antiperspirants and Deodorants", 2 Cosmetics, Science and Technology, 373-416 (M. Balsam and E. Sagarin ed. 1972). Such compositions can be generally classified as: antiperspirants, which serve to stop or reduce flow of perspiration; perfumes, which mask any objectionable odors resulting from perspiration; and deodorants, which stop or reduce the production of malodorous material in perspiration. For a variety of reasons, deodorant compositions are preferred by a large number of consumers. For example, some individuals are unable to use commercially-available antiperspirant products due to hypersensitivity to the astringent materials typically used in those products.

Deodorant compositions have primarily been thought of in the art in the context of stopping or reducing the formation of bacterial by-products in perspiration. This has been accomplished through the use of anti-microbial agents which attack the bacteria responsible for producing the malodorous by-products. While these anti-microbial deodorants have been effective in reducing malodor, they have not completely eliminated the formation of the malodorous condition. Thus, it is desirable to provide deodorant compositions which can further reduce malodor.

Additionally, it is desirable to reduce malodor once it is formed. In certain instances, malodor say remain, e.g., in the axilla and crotch areas, or in axilla and crotch areas of articles of clothing, even after washing. Traditionally, malodor that is already formed has been masked by perfumes. It is desirable to provide improved methods and compositions for reducing malodor that can remain subsequent to washing of the person or articles of clothing. In one approach, described in PCT International Patent Application Publication Number WO 87/04341, published July 30, 1987, it is disclosed that axillary malodor can be neutralized by the use of cupric sulfate, silver sulfate, potassium permanganate, ferric chloride, sodium hydroxide, silver proteinate, sodium hypochlorite, zinc sulfate, or copper gluconate. However, it is desirable to provide alternative technology for neutralizing malodor.

It is an object of this invention to provide compositions effective for controlling nonmicrobial malodor from human sweat. It is also an object of this invention to provide methods effective for controlling nonmicrobial malodor from human sweat.

## SUMMARY OF THE INVENTION

The present invention provides compositions containing a thiol heterocyclic compound and a topical carrier for topical application to the skin in the axilla or crotch region or to articles of clothing which are worn in the vicinity of such skin. The applicable deodorant agents comprise thiol heterocyclic compounds, or a pharmaceutically-acceptable salt thereof, comprising a heterocyclic ring structure with from 5 to 10 heterocyclic ring atoms, wherein 1 to 3 of said ring atoms are selected from the group consisting of oxygen, nitrogen, and sulfur, and the remaining heterocyclic ring atoms being carbon atoms; and wherein said heterocyclic ring has at least 1 thiol substituent attached to a carbon atom of said heterocyclic ring.

## DETAILED DESCRIPTION OF THE INVENTION

Although it has been suggested that malodor formation and neutralization may involve more than one mechanism, such mechanisms are not completely understood. Applicant has determined that even when perspiration from the axilla or crotch areas of humans is sterilized such that substantially no live bacteria remain to produce malodorous bacterial by-products, the perspiration can still develop aesthetically objectionable malodor. Such malodor shall hereinafter be referred to as "nonmicrobial malodor". Applicant has determined that certain vitamin $B_6$ compounds commonly secreted by humans from their eccrine sweat glands react when mixed with apocrine sweat gland secretions to form nonmicrobial malodor. Applicant has further determined that deodorant compositions containing certain thiol heterocyclic compounds are highly effective for reducing such nonmicrobial malodor in human perspiration. These compositions are particularly useful for controlling malodor in perspiration containing both apocrine and eccrine sweat. Furthermore, such compositions are effective for reducing nonmicrobial malodor rapidly after such odor is formed, or preventing nonmicrobial malodor.

Thiol heterocyclic compounds of the type described herein have been found to be highly effective for reducing the occurrence of the malodorous perspiration and, further, for deodorizing malodorous perspiration. Nonmicrobial malodor increases over time, especially when the rate of perspiration is high and when the time between washing is extended. It is believed that the concentration of nonmicrobial malodor precursors on the skin increases as additional perspiration is secreted and the aqueous component of previously secreted perspiration evaporates, thus increasing the formation of discernible nonmicrobial malodor. Without being limited by any theory, it is believed that thiol heterocyclic compounds react with the odor molecules produced from the interaction of the aldehyde form of vitamin $B_6$ (PLP) with sweat to reduce the volalitity of the odorants and thereby reduce their ability to reach the human olfactory organ. The occurrence of such malodorous conditions from nonmicrobial sources can be substantially diminished by the thiol heterocyclic compounds of the present invention.

The compositions of the present invention comprise, as essential components, a thiol heterocyclic compound for controlling nonmicrobial malodor and a compatible carrier. As used herein, "controlling nonmicrobial malodor" means reducing previously formed nonmicrobial malodor to a less perceptable level to the human olfactory senses and/or inhibiting the occurrence of nonmicrobial malodor. As used herein, "compatible" means that none of the components of the carrier reacts with the thiol heterocyclic compound such that the ability of the composition to control nonmicrobial malodor is substantially impaired.

The term "alkyl", as used herein, means carbon-containing chains that are straight, branched or cyclic; and which are saturated, monounsaturated (i.e. one double or triple bond in the chain) or polyunsaturated (e.g. two or more double bonds in the chain; two or more triple bonds in the chain; one or more double and one or more triple bonds in the chain) which are substituted or unsubstituted. As used herein, saturated alkyl groups are referred to as "alkanyl"; unsaturated alkyl groups comprising double bonds bonds in the chain are referred to as "alkenyl"; and unsaturated alkyl groups comprising triple bonds in the chain are referred to as "alkynyl". The term "short chain alkyl", as used herein, means alkyls having from 1 to about 6 carbon atoms in the chain.

The term "aryl", as used herein, means aryl radicals which are substituted or unsubstituted. "Substituted aryl" means aryl radicals which have substituents on the aryl ring. Examples of aryls include phenyl, napthyl, and substituted phenyl or napthyl.

The term "substituted", as used herein, means mono- or polysubstituted, especially mono-, di- or trisubstituted. Examples of substitutents include halogen (especially fluorine, chlorine or bromine), alkyl, hydroxy, amino, aryl (especially phenyl or napthyl), carboxylate, nitro, $-CF_3$ and -OR wherein R is an unsubstituted alkyl group having from about 1 to about 3 carbon atoms (especially methoxy and ethoxy). Alkyl substituents are preferably $C_1$-$C_6$ alkyl, more preferably methyl or ethyl.

The deodorant agents of the present invention are thiol heterocyclic compounds wherein said compounds have a heterocyclic ring structure having at least one atom in the ring structure being a nitrogen, oxygen, or sulfur atom, and have a thiol substituent on the heterocyclic ring structure. As used herein, "heterocyclic ring structure" means a covalently bonded structure comprising a ring of from about 5 to about 8 atoms which may additionally comprise one or more other rings wherein all the rings are fused in the structure. As used herein "fused" rings are rings which have two or more ring atoms in common. The heterocyclic ring structures may be saturated, unsaturated or partially or wholly aromatic. Examples of heterocyclic ring structure compounds having oxygen, nitrogen, and sulfur ring atoms are found in CRC Handbook of Chemistry and Physics, 57th edition, Robert C. Weast, ed., CRC Press, Cleveland, Ohio, pp C-35 through C-58, incorporated by reference herein.

The heterocyclic ring structures of the deodorant agents of the present invention preferably contain from about 5 to about 10 ring atoms, more preferably 5 or 6 ring atoms. Also preferred is one to three atoms of said ring atoms being independently selected from the group consisting of oxygen, nitrogen, and sulfur; more preferred is one or two of said ring atoms being selected from the group consisting of oxygen, nitrogen, and sulfur; even more preferred is one to three of said ring atoms being nitrogen; even further preferred is one or two of said ring atoms being nitrogen; and the remainder of the ring atoms being carbon. In the heterocyclic ring structures, ring heteroatoms are not bonded directly to other ring heteroatoms. The nitrogen atom(s) of said ring structure are not directly bonded to an oxygen atom to form a N-oxide.

Compositions of the present invention comprise thiol heterocyclic compounds comprising a single ring or a plurality of fused rings; preferred is said thiol heterocyclic compound comprising a single heterocyclic ring structure having a thiol substituent or a heterocyclic ring structure having a thiol substituent located on any ring of such fused ring structure. Preferably, said thiol substituent on said fused ring structure is located on a heterocyclic ring. Preferred fused ring structures have two 5- or 6-membered rings.

The thiol heterocyclic compound may have a single thiol substituent or a plurality of thiol substituents. Preferably, the thiol heterocyclic compound has fewer than about four thiol substituents, preferably one or two thiol substituents, and most preferably, one thiol substituent.

The thiol substituted heterocyclic ring structure of the thiol heterocyclic deodorant agents of the present invention may be unsubstituted (other than the thiol substituent) or further substituted. Other substituents (other than the thiol) of said heterocyclic ring structure are preferably attached to the carbon atoms of said ring structure, and are preferably selected from the group consisting of hydroxy, amino, and substituted and unsubstituted alkyl. Preferred alkyls have from 1 to about 8 carbon atoms; more preferred alkyls are $C_1$-$C_8$ alkanyls and alkenyls. The nitrogen atom(s) of said heterocyclic ring structure may be substituted but are preferably unsubstituted.

It is also preferable that at least one thiol substituent be attached to a carbon atom of a heterocyclic ring having a delocalized electron structure; examples of such carbon atoms include, but are not limited to, a carbon atom of a heteroaryl or aryl ring; a carbon atom of a heterocyclic ring that is double bonded to a nitrogen or sulfur atom of the heterocyclic ring; a carbon atom of a heterocyclic ring which is connected to a nitrogen or sulfur atom of the heterocyclic ring by conjugated double bonds; or a carbon atom of a heterocyclic ring which is either bonded to an oxygen atom of the heterocyclic ring or is double bonded to a carbon atom of the heterocyclic ring that is adjacent to an oxygen atom of the heterocyclic ring. "Connected to a nitrogen or sulfur atom by conjugated double bonds", as used above, means that the heterocyclic ring includes the following structural element:

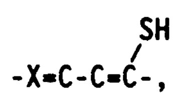

wherein X is S or N.

The oxygen atom containing heterocyclic rings, as described above, include one of the following structural elements:

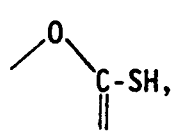

or

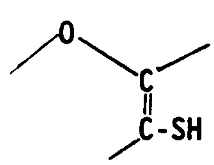

In addition to the thiol heterocyclic compounds described above, metal salts of such compounds are included in the compositions of the present invention. These include, but are not limited to, sodium, potassium, magnesium, zinc, aluminum, bismuth, and lithium salts of said compounds. Preferred are the above compounds, and their zinc, aluminum, and bismuth salts.

Preferred deodorant agents used in the composition of the present invention comprise thiol derivatives of the following classes of heterocyclic compounds: thiophenes, benzo[b]thiopenes, naphtha[2,3-6]-thiophenes, furans, pyrans, isobenzofurans, chromenes, pyrroles, imidazoles, pyrazoles, pyridines, pyrazines, pyrimidines, pyridazines, indolizines, isoindoles, indoles, purines, quinolizines, isoquinolines, quinolines, phthalazines, naphthyridines, quinoxalines, quinozolines, cinnolines, pteridines, oxazolines, isothiazoles, oxazoles, isoxazoles and furazans. Preferred classes of thiol derivative heterocyclic compounds include pyridines, pyrimidines, quinolines, and imidazoles. Especially preferred are imidazoles.

More preferred deodorant agents used in the composition of the present invention include thiol derivative heterocyclic compounds selected from the group consisting of 5,6 diamino-2-thiouracil, 1,5 diamino-6-hydroxy-2-mercaptopyrimidine, 4,5-diamino-6-hydroxy-2-mercaptopyrimidine, 2-mercaptopyrimidine, 2-thiouracil, 2,4-diamino-6-mercaptopyrimidine hemisulfate, ergothioneine, 2-thiohistidine, 2-mercaptopyridine, 4-mercaptopyridine, 2-mercaptobenzoxazole, 2-mercapto-6-nitrobenzothiazole, 2-thiocytosine, 6-thioxanthine, 2,6-dimercaptopyridine, 2-mercapto-5-nitropyridine, 2-mercaptoquinoline, 2-mercapto-4-hydroxy-6,8-dichloroquinazoline, 4-mercaptoquinazoline, 2-phenyl-4-quinazolinethiol, 3-amino-6-methyl-4-pyridazinethiol, 5-amino-4-pyridazinethiol, alpha-tocopherthiol, 2-amino-6,7-dimethyl-4-pteridinethiol, 4-mercaptopteridine, 2-mercapto-4-hydroxypteridine, and 2,4-diamino-6-phenyl-7-pteridinethiol.

Even more preferred thiol derivative heterocyclic compounds include 2-mercaptopyridine, 4-mercaptopyridine, 2-4-diamino-6-mercaptopyrimidine, 2-thiohistidine, and ergothioneine. Especially preferred are 2-thiohistidine and ergothioneine.

Thiol heterocyclic compounds applicable to this invention can be obtained commercially from industry chemical sources, such as Sigma Chemical Company (St. Louis, Missouri), Aldrich Chemical Company (Milwaukee, Wisconsin), K&K Laboratories (Plainview, New York), and P&B Research Chemicals (Waterbury, Connecticut).

It is preferable that the thiol heterocyclic deodorant agents not react with components found in perspiration in such a way that would cause the thiol heterocyclic compound to become discernibly malodorous, or which would significantly reduce its effectiveness. However, perfumes can be added to the compositions, as optional ingredients, to mask slight malodors of the thiol heterocyclic deodorant agent and its reaction products.

The compositions of the present invention comprise a safe and effective amount of a thiol heterocyclic compound when used as intended. As used herein, a "safe and effective amount" is an amount which is effective for eliminating or substantially reducing nonmicrobial malodor, while being safe for the intended use at a reasonable benefit/risk ratio.

A. Topical Compositions

One aspect of the present invention is deodorant compositions which comprise a thiol heterocyclic compound and a topical carrier. The deodorant compositions of the present invention comprising a thiol heterocyclic compound can be a variety of products which, in ordinary use, are applied topically to the skin or are applied to articles of clothing which are worn in the vicinity of the skin. Compositions formulated for topical application to skin, or prepared for deposition upon articles of clothing and intended to at least initially remain deposited at the time that the article is worn, comprise a safe and effective amount of the thiol heterocyclic compound and a topical carrier for depositing or releasing the deodorant agent. As used herein "applied to the skin" and "application to the skin" include deposit on the skin such that the deodorant agent can remain on the skin subsequent to the typical use of the composition as well as treatment of the skin with the composition wherein the deodorant agent is typically not deposited, e.g., skin cleaning compositions (discussed in more detail below) which are typically rinsed off subsequent to use.

The topical carriers of the deodorant compositions of the present invention can be in the form of liquids, solids, creams, gels, lotions, or other forms, and are preferably formulated to deposit the deodorant agent on the skin or article of clothing. As used herein, "deposited" and "deposit" of the deodorant agent on the skin means application of a deodorant composition to the skin such that the deodorant agent can remain on the skin subsequent to the typical use of the deodorant composition, including conventional post-deodorant composition application steps, if any. Such topical carriers include, but are not limited to, those formulated

as conventional deodorant compositions such as creams, sticks, roll-on liquids and spray liquids (including aerosols); body lotions, creams and oils, such as skin lotions, skin conditioners, sun and wind screens, and sun tanning lotions and oils; and skin cleansing products such as bar soaps, liquid soaps and cleaning gels.

Typically, topical deodorant compositions of the present invention contain from about 0.01% to about 20% of the thiol heterocyclic deodorant agent, preferably from about 0.05% to about 5%, more preferably from about 0.5% to about 2%.

Preferred deodorant compositions of the present invention are conventional deodorant compositions, including anti-perspirant/deodorant compositions, formulated for topical application to the axilla area of the body, or for application to the crotch area. The specific components to be included in the deodorant compositions of the present invention depend upon the particular mode of application that is desired. These methods of application, as well as the components that may be used in such compositions are well known in the art. Many such compositions are described in S. Plechner, "Antiperspirants and Deodorants" 2 Cosmetics, Science and Technology, 373-416 (M. Balsam and E. Sagarin ed. 1972), incorporated by reference herein.

A topical carrier for deodorant compositions formulated primarily for deposit of the thiol heterocyclic deodorant agent on the skin in the axilla area for malodor control Is preferably hydrophobic, with less than about 5% water, more preferably less than about 2% water, and most preferably with essentially zero percent water. Other compositions may contain high amounts of water, for the presence of water is not believed to affect the efficacy of the thiol heterocyclic deodorant agent.

Topical carriers useful for depositing the thiol heterocyclic deodorant agents to skin in the form of creams, ointments, lotions, oil-in-water and water-in-oil emulsions are known in the art, and include, for example, the water-in-oil emulsions disclosed in U.S. Patent 4,254,105, Fakuda et al., issued March 3, 1981 (incorporated by reference herein), and triple emulsion carrier systems such as the oil-in-water-in-silicone fluid emulsions as disclosed in European Patent Specification 281,394, Figueroa et al., published September 7, 1988 (incorporated by reference herein).

1. Liquids

Liquid compositions useful herein, such as roll-ons, sprays, and aerosols, preferably contain a liquid emollient as all or a substantial part of the topical carrier. Such compositions are suitable for delivery (respectively) from conventional roll-on, spray and aerosol containers known in the art. Such emollients include fatty acid and fatty alcohol esters, water-insoluble ethers and alcohols, polyorganosilicones, and mixtures thereof. Polyorganosilicones are among the preferred emollients useful herein. Liquid topical carriers are disclosed in the following patent documents, incorporated by reference herein: U.S. Patent 4,053,851, Pader, et al., October 11, 1977; U.S. Patent 4,065,564, Miles, Jr., et al., issued December 27, 1977; U.S. Patent 4,073,880, Pader, et al., issued February 14, 1978; U.S. Patent 4,278,655, Elmi, issued July 14, 1981; British Patent Application 2,018,590, Elmi, et al., published October 24, 1979; and European Patent Specification 28,853, Beckmeyer, et al., issued July 11, 1984.

The liquid deodorant compositions of the present invention may also contain an alcohol and/or a polyol as a substantial component in the topical carrier. Alcohols useful herein include ethanol, propanol, isopropanol, and mixtures thereof. Polyols useful herein include glycols such as propylene glycol. The present liquid compositions may also contain a bulking agent to modify the physical and/or cosmetic characteristics of the composition. Such bulking agents are typically present at a level of from about 1% to about 8%. Bulking agents useful herein include talc, colloidal silicas, clays, and mixtures thereof.

2. Aerosols

Aersol compositions of the present invention contain one or more volatile materials, herein "aerosol propellants", which in a gaseous state carry the other components of the present invention. The aerosol propellants useful herein typically have a boiling point within the range of from about -45°C to about 5°C. The aerosol propellants are liquified when packaged in conventional aerosol containers under pressure. The rapid boiling of the aerosol propellant upon leaving the aerosol container aids in the atomization of the other components of the aerosol compositions.

Aerosol propellants useful herein include those well known in the art. Such aerosol propellants include the chemically-inert hydrocarbons such as propane, n-butane, isobutane, cyclopropane and mixtures thereof, as well as halogenated hydrocarbons such as dichloro difluoromethane (propellant 12), 1,1-dichlor-1,1,2,2tetrafluoroethane (propellant 114), 1-chloro-1,1-difluoro-2,2trifluoroethane (propellant 115), 1-chloro-1,1-difluoroethylene (propellant 142B), 1,1-difluoroethane (propellant 152A), monochlorodifluoromethane, and mixtures thereof. Isobutane, used singly or admixed with other hydrocarbons, is preferred for use in aerosol compositions of the present invention.

3. Deodorant Sticks

Solid compositions of the present invention, as in a stick form known in the art, typically comprise a liquid base material and a solidifying agent. These deodorant sticks can generally be described as being either gel sticks or wax sticks, depending upon the particular liquid base materials and solidifying agents used. In general, liquid base materials are present at a level of from about 10% to about 97%. The solidifying agent is typically present at a level of from about 1% to about 7%.

As is appreciated by those skilled in the art, the selection of a particular liquid base material, as well as the selection of a suitable solidifying agent, will vary depending upon the particular type and rheology of deodorant stick desired. A variety of liquid base materials and solidifying agents among those useful herein, as well as sticks made from these materials, are described in the following documents, all incorporated by reference herein: S. Plechner, "Antiperspirants and Deodorants", 2 Cosmetics, Science and Technology, 373-416 (M. Balsam and E. Sagarin ed. 1972); C. Fox "Gel and Sticks Review and Update", 99 Cosmetics & Toiletries 19-52 (1984); N. Geria, "Formulation of Stick Antiperspirants and Deodorants", 99 Cosmetics & Toiletries, 55-99 (1984); and "Gels and Sticks Formulary", 99 Cosmetics & Toiletries, 77-87 (1984).

The liquid base materials used in wax deodorant sticks generally also serve as emollients, improving the cosmetic acceptability of the deodorant sticks. Such emollient materials include fatty acid and fatty alcohol esters, water-insoluble ethers and alcohols, polyorganosiloxanes, and mixtures thereof. Polyorganosiloxanes are among the preferred liquid base materials useful in wax deodorant sticks of the present invention.

Solidifying agents useful in wax deodorant sticks of the present invention are waxy materials typically incorporated at a level of from about 5% to about 50%. Among such waxy materials useful herein are the high melting point waxes, having a melting point of from about 62° C to 102° C. Lower melting point waxes having a melting point of from about 37° C to 75° C are preferred. Such low-melting point waxes include fatty acids, fatty alcohols, fatty acid esters and fatty acid amides, and mixtures thereof. Stearyl alcohol, cetyl alcohol, and mixtures thereof are among the particularly preferred waxy materials useful in the deodorant stick compositions of the present invention. Liquid base materials and solidifying agents among those useful in the wax-type deodorant sticks of this invention are disclosed in the following U.S. Patents, incorporated by reference herein: U.S. Patent 4,049,792, Elsnau, issued September 20, 1977; U.S. Patent 4,151,272, Geary, et al., issued April 24, 1979; U.S. Patent 4,299,432, Geria, issued October 21, 1980; U.S. Patent 4,280,994, Turney, issued July 28, 1981; U.S. Patent 4,126,679, Davy, et al., issued November 21, 1978; and European Patent Specification 117,070, May, published August 29, 1984.

Gel deodorant sticks of the present invention contain liquid base material which may be selected so as to also provide desirable cosmetics, such as emolliency and/or a cooling sensation when applied to the skin. Liquid base materials useful in such gel sticks include water, lower monohydric alcohols, polyhydric alcohols, and mixtures thereof. Among such materials are ethanol, isopropanol, n-propanol, n-butanol, i-butanol, t-butanol, ethylene glycol, propylene glycol, trimethylene glycol, glycerine, 1,3-butanediol, 1,4-butanediol, and mixtures thereof. Ethanol, propylene glycol, and mixtures thereof are preferred liquid base materials for use in gel sticks of this invention.

Solidifying agents useful in gel deodorant sticks of this invention are, in general, surface-active compounds which form networks immobilizing or solidifying the liquid base materials into a gel. Such solidifying agents typically include soaps, higher fatty acid amides of alkyl amines, benzylidene sorbitols, propionates and lactates, waxes, and mixtures thereof. Preferable solidifying agents in gel deodorant sticks of the present invention are nonionic in character. Among the preferred solidifying agents useful in the gel deodorant sticks of this invention are the benzylidene sorbitols, in particular the dibenzaldehyde monosorbitol acetals. Such materials are available from a variety of sources, e.g., Gell All-D™ (manufactured by New Japan Chemical Company, Ltd.) and Millithix™ (Manufactured by Milliken Chemical, Division of Milliken & Company). Liquid base materials and solidifying agents among those useful in the gel-type deodorant sticks of this invention are disclosed in the following patent documents, all Incorporated by reference herein: U.S. Patent 2,900,306, Slater, issued August 18, 1959; U.S. Patent 3,255,082, Barton, Issued June 7, 1966; U.S. Patent 4,137,306, Rubino, et al., issued January 30, 1979; U.S. Patent 4,154,816, Roehl, et al., issued May 15, 1979; U.S. Patent 4,226,889, Yuhas, issued October 7, 1980; U.S. Patent 4,346,079, Roehl, issued August 24, 1982; U.S. Patent 4,383,988, Teng, et al., issued May 17, 1983; U.S. Patent 4,504,465, Sampson et al., issued Marcy 12, 1985; and European Patent Specification 107,330,

Luebbe, et al., published May 2, 1984; and U.S. Patent 4,816,261, Luebbe et al, issued 1989. Preferred gel sticks, incorporating benzylidene sorbitols and cetyl alcohol, are described in U.S. Patent No. 4,743,444, McCall, issued May 10, 1988 (incorporated by reference herein).

The deodorant sticks of the present invention, particularly the wax-type deodorant sticks, may contain inert filler materials. Such materials include talc, colloidal silica (such as Cab-O-Sil®, sold by Cabot Corporation), clays (such as bentonites) and mixtures thereof. Such filler materials are described in U.S. Patent 4,126,679, Davy, et al., issued November 21, 1978 (incorporated by reference herein) and European Patent Specification 117,070, May, published August 29, 1984 (incorporated by reference herein).

The deodorant compositions of the present invention for topical application to skin may also contain optional components which serve as additional "active" components when deposited on the skin in addition to the thiol heterocyclic deodorant agents of the present invention. Such additional active components preferably do not, however, substantially interfere with the deodorant activity of thiol heterocyclic. Active components include, but are not limited to, other deodorant agents, such as anti-microbial agents, e.g. bacteriocides and fungicides, and antiperspirant agents. The active components must be stable in the formulations of the present compositions. A "safe and effective" amount of an active component is preferably used. Various active components among those useful in this invention are described in U.S. Patent 4,226,889, Yuhas, issued October 7, 1980 (incorporated by reference herein).

Preferred antiperspirant materials useful in the deodorant compositions of this invention include aluminum and zirconium salts, such as aluminum halides, aluminum hydroxy halides, zirconyl oxide halides, zirconyl hydroxy halides, and mixtures thereof. Antiperspirant materials among those useful herein are described in European Patent Specification 28,853, Beckmeyer, et al., published July 11, 1984 (incorporated by reference herein). Such materials are typically included at levels of from about 15% to about 40%; based upon the total weight of the composition.

Other optional antiperspirant materials include antihistamines selected from the group of ethanolamines, ethylenediamines, alkylamines, phenothiazines, and piperazines, or pharmaceutically acceptable salts thereof, as described in U.S. Patent 4,226,850, Packman, issued October 7, 1980 and U.S. Patent 4,234,566, Packman, issued November 18, 1980 (both incorporated by reference herein). Still other optional antiperspirant materials include various anticholinergic agents, such as esters of the Belladonna alkaloids scopolamine and atropine, as disclosed in U.S. Patent 3,312,709, MacMillan, issued April 4, 1967, U.S. Patent 3,326,768, MacMillan, issued June 20, 1967, U.S. Patent 3,767,786, MacMillan, issued October 23, 1973, and U.S. Patent 3,624,200, Moffett, issued November 1971 (all incorporated by reference herein).

As is appreciated by those skilled in the art, certain of the antiperspirant materials described above may be ineffective in, or lead to instability of, compositions of this invention. Accordingly, compositions of this invention may contain a buffering agent so as to maintain a pH of at least about 6.0 in the composition. Such buffering agents are described in U.S. Patent 4,154,816, Roehl, et al., issued May 15, 1979, U.S. Patent 4,346,079, Roehl, issued August 24, 1982; and U.S. Patent 4,518,582, Schamper, et al., issued May 21, 1985 (all incorporated by reference herein).

Among the preferred compositions of the present invention are those which also include a safe and effective amount of deodorant agents other than the thiol heterocyclic deodorant agent, such as antimicrobial agents (e.g. bacteriocides and fungicides), or mixtures thereof. Such other deodorant agents are usually present at levels of from about 0.1% to 10% (by weight of the composition). Suitable other deodorant agents include bacteriostatic quaternary ammonium compounds such as cetyl-trimethyl ammonium bromide, cetyl pyridinium chloride, benzethonium chloride, diisobutyl phenoxy ethoxy ethyl dimethyl benzyl ammonium chloride, sodium N-lauryl-sarcosine, sodium N-polymethyl sarcosine, lauroyl sarcosine, N-myristoyl glycine, potassium N-lauroyl sarcosine, stearyl trimethyl ammonium chloride, and mixtures thereof. Other suitable deodorant agents include 2,4,4'-trichloro-2'hydroxydiphenyl ether, zinc pyrithione (ZPT), and sodium bicarbonate. Particularly preferred other deodorant agents include a diaminoalkyl amide, such as L-lysine hexadecyl amide, as disclosed in U.S. Patent 3,574,747, Denning, issued April 13, 1971 (incorporated by reference herein).

Other optional components of the deodorant compositions of the present invention include perfumes, pigments, dyes, colorants, and ultraviolet absorbers.

The deodorant compositions of the present invention may be made by a variety of techniques well know in the art. For example, such techniques for making solid deodorant compositions are described in "Gels and Sticks Formulary", 99 Cosmetics & Toiletries 77-87 (1984), incorporated by reference herein.

4. Creams, Ointments and Lotions

Creams, ointments, and lotion composition of the present invention typically comprise one or more emollients as components of the topical carriers. As used herein, "emollients" refer to materials used for the prevention or relief of dryness, as well as for the protection of the skin. A wide variety of suitable emollients are known and may be used herein. Sagarin, Cosmetics, Science and Technology, 2nd Edition, Vol. 1, pp. 32-43 (1972), incorporated herein by reference, contains numerous examples of suitable materials. Examples of classes of useful emollients include the following:

Hydrocarbon oils and waxes; silicone oils, such as dimethyl polysiloxanes, methylphenyl polysiloxanes, water-soluble and alcohol-soluble silicone glycol copolymers; triglyceride esters, for example vegetable and animal fats and oils; acetoglyceride esters, such as acetylated monoglycerides; ethoxylated glycerides, such as ethoxylated glyceryl monostearate; alkyl esters of fatty acids having 10 to 20 carbon atoms; alkenyl esters of fatty acids having 10 to 20 carbon atoms; fatty acids having 10 to 20 carbon atoms; fatty alcohols having 10 to 20 carbon atoms; fatty alcohol ethers; ether-esters such as fatty acid esters of ethoxylated fatty alcohols; lanolin and derivatives; polyhydric alcohols and polyether derivatives; polyhydric alcohol esters; wax esters such as beeswax, spermaceti, myristyl myristate, stearyl stearate; beeswax derivatives; vegetable waxes including carnauba and candelilla waxes; phospholipids, such as lecithin and derivatives; sterols such as cholesterol and cholesterol fatty acid esters; and amides such as fatty acid amides, ethoxylated fatty acid amides, solid fatty acid alkanolamides.

Particularly useful emollients which provide skin conditioning are glycerol, hexanetriol, butanetriol, lactic acid and its salts, urea, pyrrolidone carboxylic acid and its salts, amino acids, guanidine, diglycerol and triglycerol.

A lotion of the present invention in the form of a solution typically comprises from about 0.01% to about 20%, preferably from about 0.1% to about 5%, of a thiol heterocyclic deodorant agent; from about 1% to about 20%, preferably from about 5% to about 10%, of an emollient; and from about 50% to about 90%, preferably from about 60% to about 80%, water. A cream of the present invention in the form of a solution typically comprises from about 0.01% to about 20%, preferably from about 0.1% to about 5%, of a thiol heterocyclic deodorant agent; from about 5% to about 50%, preferably from about 10% to about 20%, of an emollient, and from about 45% to about 85%, preferably from about 50% to about 75%, water.

An ointment of the present invention may comprise a simple base of animal or vegetable oils or semi-solid hydrocarbons (oleaginous). Ointments may also comprise absorption ointment bases which absorb water to form emulsions. Examples of such ointment bases include anhydrous lanolin and hydrophilic petrolatum. Emulsion ointment bases may be oil-in-water or water-in-oil emulsions. Ointment carriers may also be water soluble. Examples of such ointment carriers include components such as glycolethers, propylene glycols, polyoxyl stearates, and polysorbates. An ointment typically comprises from about 2% to about 10% of an emollient plus from about 0.1% to about 2% of a thickening agent. A more complete disclosure of thickening agents useful herein can be found in Segarin, Cosmetics, Science and Technology, 2nd Edition, Vol. 1, pp. 72-73 (1972), incorporated herein by reference.

If a topical carrier of the present invention is formulated as an emulsion, typically from about 1% to about 10%, preferably from about 2% to about 5%, of the carrier comprises an emulsifier. Emulsifiers may be nonionic, anionic or cationic. Suitable emulsifiers are disclosed in, for example, U.S. Patent 3,755,560, issued August 28, 1973, Dickert et al,; U.S. Patent 4,421,769, issued December 20, 1983, Dixon et al.; and McCutcheon's Detergents and Emulsifiers, North American Edition, pages 317-324 (1986); the disclosures of which are incorporated herein by reference. Preferred emulsifiers are anionic or nonionic, although the other types may also be used.

Single emulsion skin care preparations, such as lotions and creams, of the oil-in-water type and water-in-oil type are well-known in the cosmetic art and are useful in the present invention. Multiphase emulsion compositions, such as the water-in-oil-in-water type, as disclosed in U.S. Patent No. 4,254,105, Fakuda et al., issued March 3, 1981, herein incorporated by reference, are also useful in the present invention. In general, such single or multiphase emulsions contain water, emollients and emulsifiers as essential ingredients.

Triple emulsion carriers comprising an oil-in-water-in-silicone fluid emulsion composition as disclosed in European Patent Specification 281,394 (supra) are also useful in the present invention. More particularly, such triple emulsion carrier systems comprise a) from about 15% to about 90% by weight (of the vehicle) of a silicone fluid continuous phase consisting essentially of at least one liquid organopolysiloxane, b) from about 30% to about 80% by weight (of the vehicle) of an aqueous discontinuous phase comprising an oil-in-water emulsion of a cosmetically-acceptable oily liquid non-particulate phase dispersed in an aqueous phase and c) from about 0.5% to about 5% by weight (of the vehicle) of an effective dispersing amount of dimethicone copolyol for dispersing (b) in (a).

Another emulsion carrier useful in the compositions of the present invention is a micro-emulsion carrier. Such a carrier comprises from about 9% to about 15% squalane; from about 25% to about 40% silicone oil; from about 8% to about 20% of a fatty alcohol; from about 15% to about 30% of polyoxyethylene sorbitan mono-fatty acid (commercially available under the trade name Tweens®) or other nonionics; and from about 7% to about 20% water.

Lotions and creams can be formulated as emulsions as well as solutions. Typically such lotions in the form of emulsions comprise from about 0.01% to about 20%, preferably from about 0.1% to about 5%, of a thiol heterocyclic deodorant agent from about 1% to about 20%, preferably from about 5% to about 10%, of an emollient; from about 25% to about 75%, preferably from about 45% to about 95%, water; and from about 1% to about 10%, preferably from about 2% to about 5%, of an emulsifier. Such creams in the form of emulsions typically comprise from about 0.01% to about 20%, preferably from about 0.1% to about 5%, of a thiol heterocyclic deodorant agent; from about 1% to about 20%, preferably from about 5% to about 10%, of an emollient; from about 20% to about 80%, preferably from about 30% to about 70%, water; and from about 1% to about 10%, preferably from about 2% to about 5%, of an emulsifier.

5. Skin Cleansing

In addition to the deodorant compositions described above, the deodorant compositions of the present invention include skin cleansing deodorant compositions, which comprise a thiol heterocyclic compound and a topical carrier which includes a safe and effective surfactant for topical application to human skin. The term "safe and effective surfactant for topical application to human skin" refers to a surfactant which is not only an effective skin cleanser, but also can be used without undue toxicity, irritation, allergic response, and the like. The skin cleansing deodorant compositions of the present invention preferably contain from about 0.01% to about 20% of the thiol heterocyclic deodorant agent and from about 1% to about 90%, preferably from about 50% to about 90%, of a surfactant for topical application to human skin.

The physical form of the skin cleansing, deodorant compositions is not critical. The compositions can be, for example, formulated as toilet bars, liquids, pastes, or mousses. Toilet bars are most preferred since this is the form of cleansing agent most commonly used to wash the skin.

Those skilled in the art will recognize that active ingredients applied to skin from skin cleansing compositions may not significantly deposit on the skin due to factors such as rinsing which typically follows the application of such products. Even when significant deposit of the thiol heterocyclic on the skin is not attained, the compositions can still be highly effective for controlling nonmicrobial malodor that was previously formed in the axilla or crotch areas. Controlling nonmicrobial malodor in connection with skin cleaning compositions is especially of great benefit since malodorous skin condition can remain even after the affected axilla and crotch areas are washed with conventional skin cleansing compositions.

Any safe and effective surfactant which is compatible with the thiol heterocyclic deodorant agent can be used in the compositions of the present invention, including surfactants selected from anionic, nonionic, zwitterionic, amphoteric and ampholytic surfactants, as well as mixtures of these surfactants. Such surfactants are well-known to those skilled in the detergency art. Suitable surfactants can be found, for example, in McCutcheon's Detergents and Emulsifiers, North American Ed. pages 317-324 (1986), incorporated herein by reference. The particular surfactant is not believed to be critical to obtaining the anti-nonmicrobial malodor benefits of the present invention.

The skin cleansing deodorant compositions of the present invention can optionally contain, at their art-established levels, materials which are conventionally used in skin cleansing compositions, including antibacterial agents and fungicides, including those described above and also those described in U.S. Patent 3,256,200, Reller et al., issued June 14, 1966 (incorporated by reference herein); emollients, such as those described above, and also including mineral oils, paraffin wax having a melting point of from about 100°F to about 170°F, fatty sorbitan esters (see U.S. Patent 3,988,255, Seiden, issued October 26, 1976, incorporated by reference herein), lanolin and lanolin derivatives, esters such as isopropyl myristate and triglycerides such as coconut oil or hydrogenated tallow; free fatty acid, such as coconut oil fatty acid, preferably at levels up to about 10%, to improve the volume and quality (creaminess) of the lather produced by the compositions; and other ingredients such as perfumes, dyes, pigments, polymeric skin feel aids (such as cationic quaternized guar gum, e.g., Jaguar C-14-S, from Hoechst Celanese Corp.), humectants, thickening agents, preservatives, alkaline agents, propoxylated glycerol derivative skin conditioning agents, or other cosmetic adjuvants.

Skin cleansing compositions formulated as toilet soap bars generally comprise from about 50% to about 90% surfactant. Moisture is generally present at levels of from about 5% to about 20%. Skin cleansing compositions formulated as liquids generally comprise from about 10% to about 30% surfactant and from about 60% to about 90% water. Skin cleansing compositions formulated as pastes generally comprise from about 20% to about 60% surfactant and from about 30% to about 50% water. Pastes and liquids will also generally contain organic thickening agents such as natural gums and polymers.

Examples of soap-based toilet bar compositions are found in U.S. Patent 3,567,749, Megson et al., issued April 27, 1971, incorporated herein by reference. Examples of synthetic-based toilet bars which can be used in preparing compositions of the present invention are found in U.S. Patent 2,987,484, Lundberg et al., issued June 6, 1961, incorporated by reference herein. Other examples of soap/synthetic-based toilet bars are found in U.S. Patent 3,070,547, Chaffee, issued December 25, 1962 and U.S. Patent 3,376,229, Haas et al., issued April 2, 1967, incorporated herein by reference. Examples of soap-based liquid cleansing compositions which can be used in preparing liquid compositions of the present invention are found in U.S. Patent 4,310,433, Stiros, issued January 12, 1982, incorporated herein by reference. Examples of synthetic-based liquid cleansing compositions which can be used in preparing compositions of the present invention are found In U.S. Patents 4,338,211, Stiros, issued June 6, 1982, incorporated herein by reference. Paste compositions can be made by appropriate reduction in the levels of water in the compositions of U.S. Patents 4,310,433 and 4,338,211, incorporated by reference herein. Examples of ultra mild skin cleansing compositions which can be used in preparing compositions of the present invention can be found in U.S. Patent 4,673,525, Small et al., issued June 16, 1987, incorporated by reference herein. Examples of skin cleansing mousse compositions with ethoxylated nonionic and wholly or partially esterified polyol nonionic surfactants and also having skin conditioning ingredients such as emollients and skin moisturizers can be found in U.S. Patent 4,708,813, Snyder, issued November 14, 1987, incorporated by reference herein. In addition to disclosing examples of skin cleansing compositions of the present invention, the above incorporated patents also disclose a variety of surfactants that can be used in the compositions of the present invention, including both soap-based and synthetic detergent-based surfactants. Skin cleansing deodorant compositions of the present invention are made by incorporating a thiol heterocyclic deodorant agent in the above-identified compositions.

B. Laundry Product Compositions

Another aspect of the present invention involves laundry product compositions comprising a thiol heterocyclic deodorant agent of the present invention and a laundry product carrier. Laundry product carriers can be in liquid, granular, or solid form and include liquid and granular detergents, and wash-added, rinse-added and dryer-added substrates which may also contain other ingredients, such as fabric conditioning and/or detergent ingredients. The laundry product compositions are formulated such that the thiol heterocyclic deodorant agent either deodorizes the article of clothing during the laundry step and is washed or rinsed off (removal of odor), or is deposited on the article of clothing and remains to control nonmicrobial malodor (prevention of odor). The laundry product compositions of the present invention are believed to be especially beneficial for reducing nonmicrobial malodorous substances that become absorbed into the fabric.

Typically such laundry product compositions comprise from about 0.001% to about 20% of a thiol heterocyclic deodorant agent. Laundry product compositions formulated for removal of odor preferably comprise from about 0.001% to about 1% of a thiol heterocyclic deodorant agent, more preferably from about 0.005% to about 0.5%, more preferably still from about 0.01% to about 0.1%. Laundry product compositions formulated for prevention of odor preferably comprise from about .01% to about 10% of a thiol heterocyclic deodorant agnet, more preferably from about 0.1% to about 5%.

"Laundry product compositions", as used herein, include such compositions as liquid and granular laundry detergents, liquid and granular fabric conditioning and washer or dryer added substrates also containing fabric conditioners and/or detergent ingredients. Such compositions comprise a thiol heterocyclic agents of the present invention and typically comprise, one or more of the following components.

Detersive Surfactants: The detergent compositions of this invention will contain organic surface-active agents ("surfactants") to provide the usual cleaning benefits associated with the use of such products.

Detersive surfactants useful herein include well-known synthetic anionic, nonionic, amphoteric and zwitterionic surfactants. Typical of these are the alkyl benzene sulfonates, alkul- and alkylether sulfates, paraffin sulfonates, olefin sulfonates, amine oxides, alpha-sulfonates of fatty acids and of fatty acid esters, alkyl glycosides, ethoxylated alcohols and ethoxylated alkyl phenols, and the like, which are well-known from the detergency art. In general, such detersive surfactants contain an alkyl group in the $C_9$-$C_{18}$ range;

the anionic detersive surfactants can be used in the form of their sodium, potassium or triethanolammonium salts. Standard texts such as the McCutcheon's Index contain detailed listings of such typical detersive surfactants. $C_{11}$-$C_{14}$ alkyl benzene sulfonates, $C_{12}$-$C_{18}$ paraffin-sulfonates, and $C_{11}$-$C_{18}$ alkyl sulfates and alkyl ether sulfates are especially preferred in the compositions of the present type.

Also useful herein are the water-soluble soaps, e.g., the common sodium and potassium coconut or tallow soaps well-known in the art. Unsaturated soaps such as alkyl soaps may be used, especially in liquid formulations. Saturated or unsaturated $C_9$-$C_{16}$ hydrocarbyl succinates are also effective.

Mixtures of the anionics, such as the alkylbenzene sulfonates, alkyl sulfates and paraffin sulfonates, with $C_9$-$C_{16}$ ethoxylated alcohol surfactants are preferred for through-the-wash cleansing of a broad spectrum of soils and stains from fabric.

Combinations of anionic, cationic and nonionic surfactants can generally be used. Such combinations, or combinations only of anionic and nonionic surfactants, are preferred for liquid detergent compositions. Such surfactants are often used in acid form and neutralized during preparation of the liquid detergent composition. Preferred anionic surfactants for liquid detergent compositions include linear alkyl benzene sulfonates, alkyl sulfates, and alkyl ethoxylated sulfates. Preferred nonionic surfactants include alkyl polyethoxylated alcohols.

Anionic surfactants are preferred for use as detergent surfactants in granular detergent compositions. Preferred anionic surfactants include linear alkyl benzene sulfonates and alkyl sulfates. Combinations of anionic and nonionic detersive surfactants are especially useful for granular detergent applications.

Detergent compositions will typically contain from about 10% to about 60% of a water-soluble detergent surfactant. Suitable surfactants and detergent compositions are described in U.S. Patent 3,929,678, Laughlin et al., issued December 30, 1975 and U.S. Patent 4,294,710, Hardy et al., issued October 13, 1981, both of which are incorporated herein by reference.

Conventional Builders: Builders used in the practice of this invention include various metal ion sequestering agents such as amine chelants and phosphonate chelants, such as diethylenetriamine pentaacetates, the alkylene amino phosphonates such as ethylenediamine tetraphosphonate, and the tripolyphosphate and "pyro" builders well known in the art. Importantly, various nonphosphorus builders can be used. Included among these by way of exemplification, but not limitation, are: 1-10 micron Zeolite A; 2,2'-oxodisuccinate; tartrate mono- and di-succinates; citrates; $C_8$-$C_{14}$ hydrocarbyl succinates; sodium carbonate; and mixtures thereof. Inorganic salts such as sodium sulfate can also be present. Lists of builders useful herein can be had by reference to U. S. Patent 4,704,233, cited above.

Bleaches: Various well-known bleaching agents (especially fiber and fabric bleaches) are well known and may be used as components of the laundry product carriers. For laundry products, the sodium perborate mono- and tetra-hydrates are preferred, although the percarbonates and persulfates are also useful. Aqueous hypochlorite is also a routine additive in many laundering operations. As noted previously, bleaching agents useful as components of the compositions of the present invention are limited to those which are compatible with the thiol heterocyclic compound in the composition.

Detersive Adjuncts: The compositions herein can contain various ingredients which aid in their cleaning performance. For example, it is preferred that the laundry compositions herein also contain enzymes to enhance their through-the-wash cleaning performance on a variety of soils and stains. Amylase and protease enzymes suitable for use in detergents are well-known in the art and in commercially available liquid and granular detergents. Commercial detersive enzymes (preferably a mixture of amylase and protease) are typically used at levels of 0.001% to 2%, and higher, in the present compositions.

Moreover, the compositions herein can contain, in addition to ingredients already mentioned, various other optional ingredients typically used in commercial products to provide aesthetic or additional product performance benefits. Typical ingredients include pH regulants, perfumes, dyes, bleaches, optical brighteners, polyester soil release agents, hydrotropes and gel-control agents, freeze-thaw stabilizers, bacteriocides, preservatives, suds control agents, bleach activators and the like. Fabric softeners, especially clays and mixtures of clays with various amines and quaternary ammonium compounds, can all be used in the compositions. Such matters are well-known from the patent literature and in commercial practice.

The compositions herein are prepared using conventional techniques, well-known to the formulator of commercial detergent and bleach products.

Fabric conditioning agents, when present in the laundry product compositions, typically comprise between about 1% and about 35% of the composition, preferably between about 6% and about 25%. The particular fabric conditioning agent utilized is not believed to be critical to the present invention, and any of those fabric conditioning agents known in the art are believed to be applicable. These include, for example, quaternary ammonium fabric conditioners, such as those disclosed in U.S. Patent 3,936,537, Baskerville et al., issued February 3, 1976, tertiary amines, such as those disclosed in British Patent 1,514,276, Kenyon,

published June 14, 1978, amine-anion ion-pair complexes including those disclosed in British Patents 1,077,103 and 1,077,104, assigned to Bayer, published July 26, 1977, and U.S. Serial No. 153,172, Caswell, filed February 8, 1988, and smectite-type clay softening systems, such as those described in U.S. Patent 4,062,647, Storn et al., issued December 13, 1977, British Patent 1,483,627, assigned to Procter & Gamble, published Aubust 24, 1977.

Aqueous dispersions useful for direct application to articles of clothing in an aerosol form comprise a thiol heterocyclic deodorant agent; from about 0.1% to 10% water; from about 0.01% to about 5% of a suitable organic solvent; the balance being a suitable propellant. Examples of such propellants are the chlorinated, fluorinated and chlorofluorinated lower molecular weight hydrocarbons. Nitrous oxide, carbon dioxide, isobutane and propane may also be used as propellant gases. These propellants are used at a level sufficient to expel the contents of the container. Suitable organic materials useful as the solvent or a part of a solvent system are as follows: propylene glycol, polyethylene glycol (M.W. 200-600), polypropylene glycol (M.W. 425-2025), glycerine, sorbitol esters, 1,2,6-hexanetriol, deithyl tartrate, butanediol, and mixtures thereof. The balance of the composition comprises a liquid carrier, preferably the carrier is water or a mixture of water and monohydric alcohols.

Liquid fabric-treatment compositions can be prepared by mixing the thiol heterocyclic deodorant agent into a solvent, alone or with other components.

The deodorant agents of the present invention can be added to granular carriers by admixing the deodorant agent in solution with other of the composition's ingredients prior to the time when they are spray dried, and then spray drying said mixture in a conventional manner. Alternatively, granular or powder laundry product compositions can be made by dry blending the deodorant agents with other of the composition ingredients.

C. Fabric Treatment Compositions

Another aspect of the present invention involves fabric treatment compositions comprising thiol heterocyclic agents of the present invention for deposition onto articles of clothing or fabric worn in the vicinity of the skin, and a fabric treatment carrier. A "fabric treatment carrier", as used herein, typically comprises one or more of the following components: optical brighteners, surfactants (preferably nonionic or ionic) or organic esters. The fabric treatment compositions can, it is believed, inhibit or reduce the formation of nonmicrobial malodor due to axilla or crotch area sweat that has been absorbed by said clothing. Fabric treatment carriers include liquid and granular fabric conditioning compositions, and stain and/or odor removing compositions formulated such that the thiol heterocyclic deodorant agent is deposited on articles of clothing prior to washing or drying of said articles, or prior to use or wearing of said articles, such that the deodorant agent is deposited upon an article and remains deposited at least during the initial period when the article is worn or used. Typically, such compositions will contain from about 0.01% to about 20% of the thiol heterocyclic compounds of the present invention, preferably from about 0.1% to about 10%.

D. Catamenials and Diapers

Another aspect of the present invention involves compositions to control nonmicrobial malodor comprising an absorbent substrate carrier on a catamenial or diaper and a deodorant agent of the present invention. "Absorbent substrate carrier", as used herein, means an absorbent layer or core of material comprising wood pulp fiber, cotton fibers, polyester fibers or other liquid or solid absorbent materials. Deodorant compositions containing the deodorant agent of the present invention for use to control urine-based and menstrual fluid-based malodor can include, but are not limited to, catamenial products such as sanitary napkins and panty liners and infant and adult diapers. Another aspect of the present invention provides deodorant and deodorant/anti-septic compositions comprising the deodorant agent of the present invention and perfumes or antimicrobial agents for use in urinals and toilets, catamenials, and bedding.

E. Methods

The present invention also provides methods for controlling or inhibition of malodor, preferably malodor of human perspiration comprising topically applying a safe and effective amount of one or more of the thiol heterocyclic compositions of the present invention to areas of the skin subject to secretion of both apocrine and eccrine sweat, i.e., the axilla and crotch areas of the body.

Methods of using the composition of the present invention to control nonmicrobial malodor comprise topically applying compositions of the present invention; the compositions are preferably applied such that from about 0.002 mg to about 4.0 mg, preferably from about 0.01 mg to about 1.0 mg, of the deodorant agent is applied per one square centimeter of skin.

Additionally, the present invention provides methods of controlling nonmicrobial malodor in articles of clothing and fabric by depositing a safe and effective amount of a thiol heterocyclic agent of the present invention to an area of the article of clothing of fabric. Preferably the deodorant composition is applied to the article of clothing at areas which typically come in contact with both eccrine gland and apocrine gland perspiration when worn, particularly the axilla and crotch regions. Preferably, from about 0.002 to about 4.0, preferably from about 0.01 to about 1.0, milligrams of the thiol heterocyclic deodorant agent is deposited per one square centimeter of the article of clothing or fabric.

The present invention also provide methods of contacting an article of clothing or fabric with an aqueous detergent solution comprising from about 0.1% to about 2% by weight of a detergent composition of the present invention. Fabrics to be laundered are agitated, preferably in an automatic washing machine, in these solutions to effect cleaning, stain removal, fabric care benefits, and malodor control. Compositions can also be applied to articles of clothing or fabric by tumbling said articles with the composition in an automatic laundry dryer.

The present invention also provides methods of controlling malodor comprising contacting or depositing a thiol heterocyclic deodorant agents of the present invention to catamenial products that come into contact with urine, feces and/or menstrual fluid. Preferred methods further comprising contacting or depositing the deodorant agents to catamenial products comprising an absorbent substrate carrier.

The following non-limiting examples illustrate compositions of the present invention.

## EXAMPLES

### Examples I - XXIV

Deodorant compositions of a gel-type solid form are made comprising the ingredients listed in Tables I - IV.

Table I

| Ingredient | I | II | III | IV | V | VI |
|---|---|---|---|---|---|---|
| 2,4-diamino-6-mercapto pyrimidine | 0.5 | - | - | - | - | - |
| 2-thiouracil | - | 0.5 | - | - | - | - |
| 4,5-diamino-6-hydroxy-2-mercaptopyrimidine | - | - | 0.5 | - | - | - |
| 2-mercaptobenzoxazole | - | - | - | 0.5 | - | - |
| 2-mercaptopyridine | - | - | - | - | 0.5 | - |
| 2-thiohistidine | - | - | - | - | - | 0.5 |
| ethanol | 5.0 | 5.0 | 5.0 | 5.0 | 5.0 | 5.0 |
| EDTA [1] | 1.0 | 1.0 | 1.0 | 1.0 | 1.0 | 1.0 |
| water | 93.5 | 93.5 | 93.5 | 93.5 | 93.5 | 93.5 |

[1]: ethylenediamine tetraacetic acid, chelating agent

Table II

| Ingredient | VII | VIII | IX | X | XI | XII |
|---|---|---|---|---|---|---|
| 2,4-diamino-6-mercapto pyrimidine | 3.0 | - | - | - | - | - |
| 2-thiouracil | - | 3.0 | - | - | - | - |
| 4,5-diamino-6-hydroxy 2-mercaptopyrimidine | - | - | 3.0 | - | - | - |
| 2-mercaptobenzoxazole | - | - | - | 3.0 | - | - |
| 2-mercaptopyridine | - | - | - | - | 3.0 | - |
| 2-thiohistidine | - | - | - | - | - | 3.0 |
| ethanol | 41.0 | 41.0 | 41.0 | 41.0 | 41.0 | 41.0 |
| triclosan* | 0.3 | 0.3 | 0.3 | 0.3 | 0.3 | 0.3 |
| cetyl alcohol | 5.0 | 5.0 | 5.0 | 5.0 | 5.0 | 5.0 |
| propylene glycol | 41.4 | 41.4 | 41.4 | 41.4 | 41.4 | 41.4 |
| cyclomethicone (D5[2]) | 5.0 | 5.0 | 5.0 | 5.0 | 5.0 | 5.0 |
| dibenzaldehyde monosorbitol acetal | 3.0 | 3.0 | 3.0 | 3.0 | 3.0 | 3.0 |
| colorants | 1.3 | 1.3 | 1.3 | 1.3 | 1.3 | 1.3 |

[2]: cyclomethicone D5 supplied by Dow Corning Corp.
*: triclosan supplied by Aldrich Chemical Company

Table III

| Ingredient | XIII | XIV | XV | XVI | XVII | XVIII |
|---|---|---|---|---|---|---|
| 2,4-diamino-6-mercapto pyrimidine | 1.0 | - | - | - | - | - |
| 2-thiouracil | - | 1.0 | - | - | - | - |
| 4,5-diamino-6-hydroxy 2-mercaptopyrimidine | - | - | 1.0 | - | - | - |
| 2-mercaptobenzoxazole | - | - | - | 1.0 | - | - |
| 2-mercaptopyridine | - | - | - | - | 1.0 | - |
| 2-thiohistidine | - | - | - | - | - | 1.0 |
| cyclomethicone (D5) | 44.0 | 44.0 | 44.0 | 44.0 | 44.0 | 44.0 |
| macrospherical ZAG complex [3] | 26.7 | 26.7 | 26.7 | 26.7 | 26.7 | 26.7 |
| stearyl alcohol | 11.0 | 11.0 | 11.0 | 11.0 | 11.0 | 11.0 |
| Fluid AP [4] | 5.0 | 5.0 | 5.0 | 5.0 | 5.0 | 5.0 |
| talc | 6.5 | 6.5 | 6.5 | 6.5 | 6.5 | 6.5 |
| fragrance | 0.8 | 0.8 | 0.8 | 0.8 | 0.8 | 0.8 |

[3]: zirconium-aluminum-glycine hydroxy chloride complex antiperspirant material

[4]: water insoluble ether, condensation product of about 14 moles propylene oxide with one mole butyl alcohol, sold by Union Carbide Corporation

Table IV

| Ingredient | XIX | XX | XXI | XXII | XXIII | XXIV |
|---|---|---|---|---|---|---|
| 2,4-diamino-6-mercapto pyrimidine | 3.0 | - | - | - | - | - |
| 2-thiouracil | - | 3.0 | - | - | - | - |
| 4,5-diamino-6-hydroxy 2-mercaptopyrimidine | - | - | 3.0 | - | - | - |
| 2-mercaptobenzoxazole | - | - | - | 3.0 | - | - |
| 2-mercaptopyridine | - | - | - | - | 3.0 | - |
| 2-thiohistidine | - | - | - | - | - | 3.0 |
| ethanol | 46.8 | 46.8 | 46.8 | 46.8 | 46.8 | 46.8 |
| propylene glycol | 46.9 | 46.9 | 46.9 | 46.9 | 46.9 | 46.9 |
| dibenzaldehyde monosorbitol acetate | 3.3 | 3.3 | 3.3 | 3.3 | 3.3 | 3.3 |

The compositions, comprised as above, are made by admixing the components. The mixture is heated to approximately 88°C and stirred. The mixture is then poured into stick-forms and solidified rapidly upon cooling.

Examples XXV - XXXVI

Deodorant compositions of a gel-type solid are made comprising the ingredients listed in Tables V and VI.

Table V

| Ingredient | XXV | XXVI | XXVII | XXVIII | XXIX | XXX |
|---|---|---|---|---|---|---|
| 2,4 diamino-6-mercapto pyrimidine | .01 | .1 | 1.0 | 5.0 | 10.0 | 20.0 |
| dipropylene glycol | 28.4 | 28.4 | 28.4 | 28.4 | 28.4 | 28.4 |
| myristyl ether | 21.0 | 21.0 | 21.0 | 21.0 | 21.0 | 21.0 |
| cyclomethicone (D5) | 19.8 | 19.7 | 18.8 | 14.8 | 9.8 | 4.8 |
| sodium stearate | 6.0 | 6.0 | 6.0 | 6.0 | 6.0 | 6.0 |
| stearic acid | 0.5 | 0.5 | 0.5 | 0.5 | 0.5 | 0.5 |
| ethanol | 24.0 | 24.0 | 24.0 | 24.0 | 24.0 | 24.0 |
| triclosan | 0.3 | 0.3 | 0.3 | 0.3 | 0.3 | 0.3 |

Table VI

| Ingredient | XXXI | XXXII | XXXIII | XXXIV | XXXV | XXXVI |
|---|---|---|---|---|---|---|
| 2,4 diamino-6-mercapto pyrimidine | .01 | .1 | 1.0 | 5.0 | 10.0 | 20.0 |
| dipropylene glycol | 28.4 | 28.4 | 28.4 | 28.4 | 28.4 | 28.4 |
| myristyl ether | 21.0 | 21.0 | 21.0 | 21.0 | 21.0 | 21.0 |
| cyclomethicone (D5) | 19.8 | 19.7 | 18.8 | 14.8 | 9.8 | 4.8 |
| sodium stearate | 6.0 | 6.0 | 6.0 | 6.0 | 6.0 | 6.0 |
| stearic acid | 0.5 | 0.5 | 0.5 | 0.5 | 0.5 | 0.5 |
| ethanol | 24.0 | 24.0 | 24.0 | 24.0 | 24.0 | 24.0 |

The compositions, comprised as above, are made by admixing the components. The mixture is heated to approximately 88°C and stirred. The mixture is then poured into stick-forms and solidified rapidly upon cooling.

EXAMPLE XXXVII

A liquid deodorant comprised as below is prepared.

| Component | % (by weight) |
|---|---|
| 2-thiohistidine | 1.0 |
| triclosan | 0.5 |
| water | 92.5 |
| ethanol | 5.0 |
| EDTA | 1.0 |

The above components are admixed and placed in a conventional pump-spray apparatus. The composition can be sprayed directly onto axilla and crotch areas of the skin, or onto axilla and crotch areas of articles of clothing.

EXAMPLE XXXVIII

A deodorant composition in aerosol form, according to the present invention, is made comprising:

| Component | % (by weight) |
|---|---|
| 2-thiohistidine | 1.0 |
| triclosan | 0.4 |
| ethanol | 14.8 |
| propylene glycol | 14.8 |
| isobutane (propellant) | 70.0 |

An aerosol deodorant, comprised as above, is made by admixing the 2-thiohistidine, triclosan, ethanol, and propylene glycol and placing the mixture in a conventional aerosol can. The propellant is then added, under pressure, and the can sealed.

EXAMPLE XXXVIX

A liquid deodorant composition for topical application to the skin with a pump spray bottle is made comprising:

| Component | % (by weight) |
|---|---|
| glycerol | 49.8 |
| ethanol | 49.8 |
| triclosan | 0.3 |
| 4,5 diamino-6-hydroxy-2-mercaptopyrimidine | 0.1 |

The liquid deodorant is prepared by admixing the components noted above, adjusting the mixture to pH 9.0 with NaOH, and placing the mixture in a conventional pump spray bottle.

EXAMPLE XXXX

A liquid deodorant composition for topical application to the skin with a roll-on bottle is made comprising:

| Component | % (by weight) |
|---|---|
| glycerol | 49.8 |
| ethanol | 49.8 |
| triclosan | 0.3 |
| 2-thiohistidine | 0.1 |

The liquid deodorant is prepared by admixing the components noted above, adjusting the mixture to pH 9.0 with NaOH, and placing the mixture in a conventional roll-on bottle.

EXAMPLE XXXXI

The purpose of this example is to exemplify the effectiveness of the present invention for neutralizing and inhibiting non-microbial malodor.

A variety of thiol heterocycllc deodorant agents were added to the concentrated artificial sweat in in vitro tests. Sterile concentrated artificial sweat was prepared containing an aqueous pyridoxal phosphate solution and an aqueous solution containing natural apocrine gland secretion. The concentrated artificial sweat for each of the tests was prepared according to the following procedure, though not necessarily at the same time for each of the tested deodorant agents. The aqueous pyridoxal phosphate solution contained 12.4 mg pyridoxal phosphate per 10 ml of 20 mM tris(hydroxymethyl) amino methane, adjusted to pH 7.5 with HCl.

Natural human apocrine gland secretions were collected by firmly rolling the ball of a conventional deodorant roll-on bottle against the skin in the axilla region for at least six known malodor substrate producers for one minute each. The roll-on bottles, prior to collection of apocrine secretions, each contained 10 ml of 20 mM of tris(hydroxymethyl) amino methane adjusted to pH 7.5 with HCl. The apocrine gland secretion solutions from each bottle were then mixed together and sterilized by filtration through a 0.22 micron acetate membrane and then heat treated for 30 minutes at 80 ° C. The apocrine gland secretion was sterilized to eliminate the occurrence of microbial malodor during the test.

The artificial sweat composition was prepared by mixing one volumetric part of the pyridoxal phosphate solution with five volumetric parts of the solution containing apocrine gland secretion, under sterile conditions.

The thiol heterocyclic deodorant agents were added to 6.0 ml samples of artificial sweat composition at 0.7 mM concentration under sterile conditions and incubated for 48 hours at 37 ° C.

At the end of the incubation period, the sweat samples were organoleptically evaluated by sniffing the contents and comparing the smell to a control sample of said sweat which was incubated during the same period and under the same conditions as the deodorant agent-containing samples, but did not contain any thiol heterocyclic deodorant agent. A strong malodor was observed at the end of the incubation period for the control samples.

The following thiol heterocyclic compounds were tested at 0.7 mM concentrations in the artificial sweat (corresponding approximate gram amounts added are indicated in parenthesis): 2,4-diamino-6-mercapto pyridine (0.7 mg); 2-thiouracil (.6 mg); 4,5-diamino-6-hydroxy-2-mercaptopyrimidine (0.8 mg); 2-mercaptobenzoxazole (.8mg); 2-mercaptopyridine (0.6 mg); and L-thiohistidine (0.9 mg). In each case, the thiol heterocyclic compound substantially reduced the occurrence of nonmicrobial malodor relative to the corresponding control sample.

It was also observed that malodor from a sweat sample prepared substantially the same way as the control samples above was significantly reduced, i.e., neutralized, by the addition of 4,5-diamino-6-hydroxy-2-mercapto pyridine at 0.7 mM concentrations in the sweat sample subsequent to incubation.

EXAMPLE XXXXII

A granular detergent composition for household laundry use is as follows:

| Component | Amount(%) |
|---|---|
| Sodium $C_{14}$-$C_{15}$ alkylsulfate | 13.3 |
| Sodium $C_{13}$ linear alkyl benzene sulfonate | 5.7 |
| $C_{12}$-$C_{13}$ alkylpolyethoxylate (6.5) | 1.0 |
| Sodium toluene sulfonate | 1.0 |
| *TMS/TDS, sodium salt, 86/14 weight ratio of TMS:TDS | 25.0 |
| Sodium N-hydroxyethylethylenediaminetriacetate | 2.0 |
| Sodium polyacrylate (Avg. M.W. approx. 5000) | 2.0 |
| Sodium carbonate | 20.3 |
| Sodium silicate | 5.8 |
| Polyethylene glycol (Avg. M.W. approx. 8000) | 1.0 |
| 2-thiohistidine | 1.0 |
| Sodium sulfate, water and miscellaneous** | 29.1 |
| | 100.0 |

*TMS/TDS - mixture of tartrate monosuccinate and tartrate disuccinate in a TMS to TDS weight ratio of 85/15 sodium salt free

**includes perfume, buffers, colorants, opacifiers and the like

The components are added together with continuous mixing with sufficient extra water (about 40% total) to form an aqueous slurry which is then spray dried to form the composition. Ergothioneine or other thiol heterocyclic compounds useful in the composition of the present invention may be substituted for the above thiol heterocyclic compounds in the composition.

EXAMPLE XXXXIII

A liquid detergent composition for household laundry use is as follows:

| Component | Amount(%) |
|---|---|
| Potassium $C_{14}$-$C_{15}$ alkyl polyethoxy (2.5) sulfate | 8.3 |
| $C_{12}$-$C_{14}$ alkyl dimethyl amine oxide | 3.3 |
| Potassium toluene sulfonate | 5.0 |
| Monoethanolamine | 2.3 |
| TMS/TDS triethanolamine salt, 85/15 TMS/TDS | 15.0 |
| Potassium salt of 1,2-dihydroxy-3,5-disulfobenzene | 1.5 |
| Potassium polyacrylate (avg. M.W. approx. 9000) | 1.5 |
| 2-thiohistidine | 1.0 |
| Water and miscellaneous* | 62.1 |
| | 100.0 |

*includes perfume, buffers, colorants, opacifiers and the like

The components are added together with continuous mixing to form the composition. Ergothioneine or other thiol heterocyclic compounds useful in the composition of the present invention may be substituted for the above thiol heterocyclic compounds in the composition.

EXAMPLE XXXXIV

Fabric Conditioning Agent

| Component | Amount(%) |
|---|---|
| Ditallow di-methyl ammonium chloride (DTDMAC) | 3.7 |
| Methyl-1-tallow amido ethyl 2-tallow imidazoline | 3.7 |
| 2,2'-Dipyridaldisulfide | 3.0 |
| Water and miscellaneous* | 89.6 |
| | 100.0 |

*includes perfume, buffers, colorants, opacifiers and the like

EXAMPLE XXXXV

Additional granular detergent compositions of the present invention comprise the following ingredients:

| Ingredient | Percent (wt) |
|---|---|
| Sodium 12.3 linear alkyl benzene sulfonate | 15.8 |
| Sodium $C_{14}$-$C_{15}$ alkyl sulfate | 6.8 |
| $C_{12}$-$C_{13}$ alcohol ethoxylate (EO 6) | 0.5 |
| Sodium tripolyphosphate | 6.8 |
| Sodium pyrophosphate | 13.1 |
| Sodium acid pyrophosphate | 12.4 |
| Sodium silicate (1.6 ratio $NaO/SiO_2$) | 7.6 |
| Polyethylene glycol 8000 | 0.6 |
| Sodium polyacrylate (MW 4500) | 3.4 |
| Protease enzyme* | 1.8 |
| Sodium perborate tetrahydrate | 1.9 |
| Sodium sulfate | 14.4 |
| 4,5-Diamino-6-hydroxy-2-mercaptopyrimidine | .05 |
| Balance (including water, brightener, perfume, suds suppressor) | 14.85 |
| | 100.0 |
| pH 1% aqueous solution at 20°C | 9.2 |

*Reported in Anson units per gram

Aqueous crutcher mixes of the detergent compositions are prepared and spray-dried, except for the, sodium acid pyrophosphate, enzyme, and perfume, which are admixed, so that they contain the above ingredients at the levels shown.

EXAMPLE XXXXVI

An additional granular detergent composition for household laundry use is as follows:

| Component | Amount(%) |
| --- | --- |
| Sodium $C_{14}$-$C_{15}$ alkylsulfate | 13.3 |
| Sodium $C_{13}$ linear alkyl benzene sulfonate | 5.7 |
| $C_{12}$-$C_{13}$ alkylpolyethoxylate (6.5) | 1.0 |
| Sodium toluene sulfonate | 1.0 |
| *TMS/TDS, sodium salt, 86/14 weight ratio of TMS:TDS | 25.0 |
| Sodium N-hydroxyethylethylenediaminetriacetate | 2.0 |
| Sodium polyacrylate (Avg. M.W. approx. 5000) | 2.0 |
| Sodium carbonate | 20.3 |
| Sodium silicate | 5.8 |
| Polyethylene glycol (Avg. M.W. approx. 8000) | 1.0 |
| 2,4-Diamino-6-mercaptopyrimidine | 0.05 |
| Sodium sulfate, water and miscellaneous** | 22.85 |
| | 100.0 |

*TMS/TDS - mixture of tartrate monosuccinate and tartrate disuccinate in a TMS to TDS weight ratio of 85/15 sodium salt form

**includes perfume, buffers, colorants, opacifiers and the like

The components are added together with continuous mixing with sufficient extra water (about 40% total) to form an aqueous slurry which is then spray dried to form the composition.

EXAMPLE XXXXVII

An additional liquid detergent composition for household laundry use is as follows:

| Component | Amount(%) |
|---|---|
| Potassium $C_{14}$-$C_{15}$ alkyl polyethoxy (2.5) sulfate | 8.3 |
| $C_{12}$-$C_{14}$ alkyl dimethyl amine oxide | 3.3 |
| Potassium toluene sulfonate | 5.0 |
| Monoethanolamine | 2.3 |
| TMS/TDS triethanolamine salt, 85/15 TMS/TDS | 15.0 |
| Potassium salt of 1,2-dihydroxy-3,5-disulfobenzene | 1.5 |
| Potassium polyacrylate (avg. M.W. approx. 9000) | 1.5 |
| 2-Thiohistidine | 0.01 |
| Water and miscellaneous* | 63.09 |
| | 100.0 |

*includes perfume, buffers, colorants, opacifiers and the like

The components are added together with continuous mixing to form the composition.

EXAMPLE XXXXVIII

Fabric Conditioning Agent

| Component | Amount(%) |
|---|---|
| Ditallow di-methyl ammonium chloride (DTDMAC) | 3.7 |
| Methyl-1-tallow amido ethyl 2-tallow imidazoline | 3.7 |
| 2-Mercaptopyrydine | 0.1 |
| Water and miscellaneous | 92.5 |
| | 100.0 |

The disclosed thiol heterocyclic compounds control a variety of other malodors in addition to human perspiration. Such other malodors include, but are not limited to, environmental odors and chemical odors. In addition, the disclosed thiol heterocyclic compounds control negative odors generated by or deposited on various porous and nonporous surfaces including, but not limited to, plastics, wood, hair, glass, porcelain, fabric, fibers of varying compositions, foods, and beverages. Such negative odors may be controlled by applying the thiol heterocyclic compound to said surfaces before, during or after the odor has been generated, or by incorporating the thiol heterocyclic compound into these surfaces during manufacture. As used herein, "control odors, malodors, or negative odors" means preventing, retarding, or reversing such odor formation. As used herein, "negative odor or malodor" means an odor emanating from a particular surface or manufacture which is undesirable.

Applicants have further determined that the disclosed thiol heterocyclic compounds, preferably 4,5-diamino-6-hydroxy-2-mercaptopyrimidine, 2,4-diamino-6-mercaptopyrimidine, 2-thiohistidine, 2-mercaptopyridine, and 4-mercaptopyridine, are highly effective in controlling malodors associated with a laundry cleaning product. Though the mechanism upon which this deodorization is based is unknown, it is believed to be something beyond an interaction with vitamin $B_6$ reactions. Such a surprising application is extremely useful in laundry product compositions which do not comprise a perfume, as these compositions often have negative odors emanating from their substituents including, but not limited to, solvents, enzymes, surfactants, or contaminants. In addition to deodorizing these negative odors, the thiol heterocyclic deodorant agent enhances positive odors in the laundry product, such as perfumes.

The present invention further relates to compositions effective for controlling malodors associated with chemical formulations of laundry cleaning products. Laundry product compositions with reduced negative laundry product odor comprise from about .001% to about 20% of a thiol heterocyclic compound.

The present invention further relates to methods effective for controlling malodors associated with chemical formulations of laundry cleaning products. Methods for controlling negative laundry product odors comprise inclusion of from about .001% to about 20% of a thiol heterocyclic compound in a laundry product composition.

## Claims

1. A deodorant composition for controlling malodor from perspiration characterized in that it comprises:
   a) a safe and effective amount of a thiol heterocyclic compound
      (i) wherein said compound has a heterocyclic ring structure having at least one heteroatom, preferably from 1 to 3 heteroatoms, in the ring structure selected from oxygen, nitrogen, and sulfur, preferably nitrogen, wherein preferably the nitrogen heteroatom(s) is(are) not bonded directly to an oxygen atom to form a N-oxide;
      (ii) wherein the heterocyclic ring structure has at least 1 thiol substituent attached to a carbon atom of said heterocyclic ring; and
      (iii) wherein preferably the ring structure is unsubstituted, other than thiol substituent(s), or substituted on carbon atoms of the ring structure, wherein said substituents (other than thiol) are selected from hydroxy, amino, and substituted or unsubstituted alkyl; and
   b) a topical carrier;
   whereby the composition is suitable for application to the skin or to articles of clothing worn in the vicinity of the skin.

2. A deodorant composition for controlling malodor from perspiration, according to Claim 1 characterized in that it further comprises an antimicrobial deodorant agent.

3. A deodorant composition for controlling malodor from perspiration, according to Claim 1 or 2, characterized in that the thiol heterocyclic compound is selected from heterocyclic compounds having a thiol substituted heterocyclic ring with a delocalized electron structure, where at least one thiol substituent is
   a) attached to a heterocyclic ring carbon atom that is double bonded to a heterocyclic ring nitrogen or sulfur atom,
   b) attached to a carbon atom of said heterocyclic ring that is connected to a nitrogen or sulfur atom of said heterocyclic ring by conjugated double bonds, or
   c) attached to a heterocyclic ring carbon atom which is bonded to a heterocyclic ring oxygen atom or which is double bonded to a heterocyclic ring carbon atom that is adjacent to a heterocyclic ring oxygen atom.

4. A deodorant composition for controlling malodor from perspiration, according to Claim 3, characterized in that the thiol heterocyclic compound is selected from thiol derivatives of thiophene, furan, pyran, isobenzofuran, chromene, pyrrole, imidazole, pyrazole, pyridine, pyrazine, pyrimidine, pyridazine, indolizine, isoindole, indole, purine, quinolizine, isoquinoline, quinoline, phthalazine, naphthyridine, quinoxaline, quinozoline, cinnoline, pteridine, oxazoline, isothiazole, oxazole, and isoxazole, preferably pyridine, pyrimidine, quinoline, and imidazole.

5. A deodorant composition for controlling malodor from perspiration, according to Claim 4, characterized in that the thiol heterocyclic compound is selected from 5,6 diamino-2-thiouracil, 4,5-diamino-6-hydroxy-2-mercaptopyrimidine, 2-thiouracil, 2,4-diamino-6-mercaptopyrimidine hemisulfate, ergothioneine, 2-thiohistidine, 2-mercaptopyridine, 4-mercaptopyridine, 2,mercaptobenzoxazole, 2-mercapto-6-nitrobenzothiazole, 2-thiocytosine, 6-thioxanthine, 2,6-dimercaptopyridine, 2-mercapto-5-nitropyridine, 2-mercaptoquinoline, 2-mercapto-4-hydroxy-6,8-dichloroquinazoline, 2-mercaptopyrimidine, 4-mercaptoquinazoline, 2-phenyl-4-quinazolinethiol, 3-amino-6-methyl-4-pyridazinethiol, 5-amino-4-pyridazinethiol, alpha-tocopherthiol, 2-amino-6,7-dimethyl-4-pteridinethiol, 4-mercaptopteridine, 2-mercapto-4-hydroxypteridine, and 2,4-diamino-6-phenyl-7-pteridinethiol, preferably 2-mercaptopyridine, 4-mercaptopyridine, 2,4-diamino-6-mercaptopyrimidine, 2-thiohistidine, and ergothioneine.

**6.** A method for controlling malodor from perspiration characterized in that it comprises topically applying to skin in the axilla or crotch region, a safe and effective amount of the composition of any of Claims 1-5.

**7.** A deodorant composition characterized in that it comprises:
a) a safe and effective amount of a thiol heterocyclic compound wherein said compound has a heterocyclic ring structure having at least one heteroatom in the ring structure selected from oxygen, nitrogen, and sulfur; and wherein the heterocyclic ring structure has at least 1 thiol substituent attached to a carbon atom of the heterocyclic ring; and
b) a laundry product carrier.

**8.** A deodorant composition, according to Claim 7, characterized in that the thiol heterocyclic compound is selected from thiol derivatives of pyridine, pyrimidine, quinoline and imidazole.

**9.** A deodorant composition characterized in that it comprises:
a) a safe and effective amount of a thiol heterocyclic compound wherein said compound has a heterocyclic ring structure having at least one heteroatom in the ring structure selected from oxygen, nitrogen, and sulfur; and wherein said heterocyclic ring structure has at least 1 thiol substituent attached to a carbon atom of said heterocyclic ring; and
b) a fabric treatment carrier.

**10.** A method for controlling malodor from articles of clothing characterized in that it comprises deposition of a safe and effective amount of the composition of any of Claims 8 or 9 to an article of clothing.

European Patent Office

# EUROPEAN SEARCH REPORT

Application Number

EP 90 31 1982

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 410 610 (ORREN)<br>* Claims 1,6; page 9, lines 24-27 *<br>--- | 1-10 | A 61 K 7/32<br>A 61 L 9/01 |
| X | EP-A-0 023 676 (REWO)<br>* Claims 1,2,4; example 4 *<br>--- | 1-4 | |
| A | PATENT ABSTRACTS OF JAPAN, vol. 10, no. 355 (C-388)[2411], 29th November 1986; & JP-A-61 155 302 (SANSHO SEIYAKU K.K.) 15-07-1986<br>* Abstract *<br>--- | 1-5 | |
| A | CHEMICAL ABSTRACTS, vol. 102, no. 24, June 1985, page 338, abstract no. 209142s, Columbus, Ohio, US; & JP-A-60 16 907 (POLA CHEMICAL INDUSTRIES) 28-01-1985<br>* Abstract *<br>--- | 1-5 | |
| A | GB-A-1 280 671 (BEECHAM)<br>* Claims; examples 1,4 *<br>----- | 1-5 | TECHNICAL FIELDS SEARCHED (Int. Cl.5)<br>A 61 K |

The present search report has been drawn up for all claims

| Place of search | Date of completion of the search | Examiner |
|---|---|---|
| THE HAGUE | 05-08-1991 | WILLEKENS G.E.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)